# EUROPEAN PATENT APPLICATION

(11) **EP 4 509 159 A1**
(43) Date of publication of application: **19.02.2025**
(21) Application number: 23191603.2
(22) Date of filing: 16.08.2023
(51) Int. Cl.: A61M 15/08, A61M 15/00

(54) **NOZZLE**

(71) Applicant: Haleon CH SARL, 1197 Prangins (CH)
(72) Inventor: The designation of the inventor has not yet been filed
(74) Representative: Haleon Patent Department

(57) **Abstract**

The present invention relates to improved nozzle (1) for squeeze-actuated intranasal devices for delivering substances, such as a liquid, containing medicament to the nasal cavity of a subject. The nozzle comprises a body generally having the shape of a truncated oblique cone. The base (7) of the nozzle is substantially annular having a first central axis (2). The top of the nozzle comprises a substantially circular surface (3) having a second central axis (4). The substantially circular surface and substantially annular base are parallel with each other.The outer diameter of the substantially annular base is larger than that of the outer diameter of the substantially circular surface and the second central axis of the top is parallel to and offset from the first central axis of the base such that the surface of the body smoothly tapers from the base to the top.

## Description

### Field of the Invention

The present invention relates to improved nozzles for squeeze-actuated intranasal devices.

### Background of the Invention

The use of nasal spray devices to deliver aerosolised liquids and powders into the nose of a subject is well known. Generally, nasal sprays comprise a small bottle or container containing a liquid or powder, such as a medicament for example, and a nozzle.

Many nasal sprays comprise a pressurised container that releases the contents when the user depresses the nozzle with a downward force. However, the action required to depress the nozzle requires higher forces and may cause the nozzle to slip or move during use resulting in incorrect application or even injury. Other nasal sprays may comprise non-pressurised containers, requiring a user to squeeze the container to release the container contents. Again, given the nature of the device, a user may position the nozzle incorrectly resulting in sub-optimal administration to the wrong part of the nose and in many cases subsequent swallowing of the contents as it reaches the throat. These issues may be further compounded by differences in the ability of different users to squeeze the container.

An improved nozzle for an intranasal dispenser is disclosed in WO 20041108197.

Despite improvements, many users still don't realise that they're incorrectly using nasal sprays leading to waste, incorrect or ineffective dosing or risk of injury to the nose. Thus, there remains a need for further innovation in this area to mitigate these challenges, particularly in relation to squeeze-actuated intranasal dispensers.

### Summary of the Invention

In a First Aspect, there is provided a nozzle for intranasal administration of a liquid from a squeeze-actuated device, comprising: a body having the shape of a truncated oblique cone having a substantially annular base with a first central axis and a substantially circular surface at the top with a second central axis; an outlet positioned at the top of the body within the substantially circular surface; a channel traversing the nozzle defining a passageway in fluid communication with the substantially annular base and the outlet; wherein the outer diameter of the substantially annular base is larger than the outer diameter of the substantially circular surface and the second central axis of the top is parallel to and offset from the first central axis of the base and the surface of the body smoothly tapers from the base to the top; wherein the body is bilaterally symmetrical about a plane of symmetry the body comprising a longest generatrix and a shortest generatrix being coplanar with each other and the plane of symmetry; and wherein the shortest generatrix is a substantially straight line parallel with the second central axis and the longest generatrix is an inclined concave line such that the surface of the nosepiece smoothly transitions from a substantially flat to a concave profile around the second central axis.

Particularly the substantially annular base comprises a shoulder configured to abut against a surface of a container. In certain embodiments, the shoulder configured to abut against a surface of a container is a surface of a downwardly projecting annular rim. In certain embodiments, the shoulder configured to abut against a surface of a container is a surface of an annular flange that extends radially outwards in a direction substantially perpendicular to the first central axis.

Particularly, the nozzle further comprises a substantially cylindrical skirt extending downwardly from the substantially annular base, configured for attachment to a container. In some embodiments, the substantially cylindrical skirt extends downwardly from a position internal of the shoulder and is configured for engagement inside the neck of a container. In some embodiments, the substantially cylindrical skirt comprises at least one annular gripping rib on its outer surface for sealing and/or gripping engagement with an inner surface of the neck of a container.

Particularly, the transition between the surface of the nosepiece and the substantially circular surface is a chamfered edge.

In some embodiments, the outlet is coaxial with the second axis of the substantially circular surface. In other embodiments, the outlet is offset from the second axis of the substantially circular surface.

Particularly, the nozzle comprises a substantially circular raised lip extending upwardly from the substantially circular surface around the outlet.

Particularly, the nozzle comprises a dip tube having a liquid inlet and a liquid exit, depending from the outlet for conducting liquid upwardly from a container to the outlet.

In certain embodiments, the nozzle may further comprise at least one air channel defining a passageway for directing air from within the body, or from the top of an attached container, to at least one orifice in fluid communication with the liquid exit and the outlet, wherein in use air directed from within the body mixes with liquid being conducted upwardly from the container. Particularly, the at least one orifice is disposed about the dip tube liquid exit. In some embodiments, the nozzle comprises two air channels and two orifices positioned in a circumferential arrangement around the dip tube. Particularly, the air emitted from the orifice(s) is emitted at an angle of from 0 to 90 degrees with respect to the direction of movement of the liquid towards the outlet. In certain embodiments, an inner wall of the at least one air channel is defined and bounded by an outer wall of the dip tube.

In a Second Aspect of the Invention, there is provided a squeeze-actuated device for intranasal administration of a liquid comprising: a container defining an internal volume intended to hold the liquid, the container having at least one flexible wall and a nozzle according to the First Aspect.

In some embodiments, there is provided a squeeze-actuated device for intranasal administration of a liquid comprising: a container defining an internal volume intended to hold the liquid, the container having at least one flexible wall and a neck having an opening with an inner surface; and a nozzle according to the First Aspect wherein engagement of the outer surface of the substantially cylindrical skirt with the inner surface of the neck forms a friction-fit connection between the container and the nozzle.

Particularly, the squeeze-actuated device further comprises a liquid. More particularly the liquid is a liquid formulation of an active pharmaceutical ingredient. In certain embodiments, the liquid is saline. In certain embodiments, the liquid is a liquid formulation comprising an imidazoline nasal decongestant. In certain embodiments the liquid formulation comprises Xylometazoline or Oxymetazoline. Preferably, the liquid formulation comprises Oxymetazoline.

### Brief description of Figures

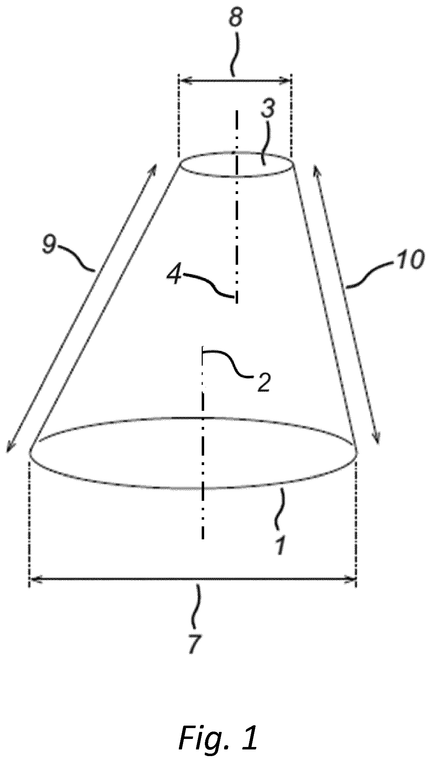

**Figure 1** provides a cartoon of the generalised shape of the nozzle (frustrum of an oblique cone). The base of the nozzle (1) is substantially annular having a central axis (2). The top of the nozzle comprises a substantially circular surface (3) also having a central axis (4). The outer diameter of the substantially annular base (7) is larger than that of the outer diameter of the substantially circular surface (8) and the second central axis of the top (4) is parallel to and offset from the first central axis of the base (2) and the surface of the body tapers from the base to the top. The nozzle body is bilaterally symmetrical about a plane of symmetry comprising a longest generatrix (9) and a shortest generatrix (10) being coplanar with each other and the plane of symmetry.
**Figure 2** illustrates a perspective view of an embodiment of the nozzle comprising an annular flange (13) and a substantially cylindrical skirt (14) further comprising an annular gripping rib (15).
**Figure 3** illustrates a perspective view of an embodiment of the nozzle that comprises a substantially circular raised lip (17) extending upwards from the substantially circular top surface (3).
**Figure 4(a) and (b)** illustrate sectional and plan views of the nozzle of Figure 3.
**Figure 5** provides a perspective sectional view of the nozzle of Figure 3.
**Figure 6** illustrates a perspective view of a further embodiment of the nozzle without an annular flange.
**Figure 7** illustrates a perspective view of an embodiment of the nozzle lacking an annular flange and that comprises a substantially circular raised lip (17) extending upwards from the substantially circular top surface (3).
**Figure 8(a) and (b)** illustrate sectional and plan views of the nozzle of Figure 7.
**Figure 9** provides a perspective sectional view of the nozzle of Figure 7.
**Figures 10(a)** **and** **(b)** show an embodiment of the nozzle in use wherein the nozzle comprises two air channels and two orifices positioned in a circumferential arrangement (at 180°) around the dip tube nozzle. Air is directed from within the nozzle body and/or headspace of a container where it mixes with liquid being conducted upward from the container. A closeup view of the interior of the nozzle is provided showing the direction of air flow and liquid and the angle (a) at which air is emitted from the air channel into the liquid as it moves towards the outlet.

### Description of the Invention

For effective nasal delivery, a spray should be directed towards the side of the nose, not at the septum. In order to do so, nozzles in the prior art need to be angled backwards and to the side of the nostril of a human user so that the dispensing path is oriented optimally. However, since this generally feels unpleasant, in the absence of proper instruction, users direct the nozzle upward, resulting in the spray being dispensed into the front region of the inner nasal space.

The present Inventors have developed a nozzle that incorporates positional cues to subconsciously communicate proper position and alignment to the user. Squeeze-actuated nasal sprays of the prior art are frequently actuated using an ipsilateral hand technique (using the hand on the same side of the body as the nostril) that can also lead to mishandling and nose bleeds (Ganesh, V., Banigo, A., McMurran, A., Shakeel, M., & Ram, B. (2017) The journal of Laryngology & Otology, 131(11), 991-996). In contrast to prior art devices, the improved nozzle of the Invention may facilitate the use of a contralateral spray technique to overcome this issue. In addition, the improved nozzle comprises features that prevent it from being inserted too far into the nostril whilst also stabilising and holding the nozzle in place during squeeze-operated dispensing, thereby avoiding accidental injury. The improved nozzle also enhances user comfort by lacking angled edges and pointed surfaces. Surprisingly, the nozzle lacks moving parts and is also easy to manufacture as a single piece by injection moulding.

The nozzle of the Invention is for use in intranasal administration of a liquid from a squeeze-actuated container, such as a bottle. Squeeze-actuation is known in the art where manual squeezing of a container formed from resiliently deformable material brings generally opposing portions of the container sidewalls closer together, causing an increase in interior pressure. This increase in interior pressure forces liquid out of the container into the nasal cavity, via the nozzle. For the avoidance of doubt, squeeze-actuated containers are non-pressurised containers, free of propellants, propellant gases and compressed gases, such as those used in aerosols for example.

The nozzles of the Invention comprise a body generally having the shape of a truncated oblique cone (frustrum of an oblique cone) - see Figure 1 for a generalised cartoon highlighting features referred to below. The base of the nozzle (1) is substantially annular having a central axis (2) referred to as the first central axis. The top of the nozzle comprises a substantially circular surface (3) also having a central axis (4), referred to as the second central axis. The terms "substantially annular" and "substantially circular" as used herein include round or circular shapes but may also encompass elliptical shapes that are not perfectly circular. The substantially circular surface and substantially annular base are parallel with each other. The outer diameter of the substantially annular base (7) is larger than that of the outer diameter of the substantially circular surface (8) and the second central axis (4) of the top is parallel to and offset from the first central axis (2) of the base such that the surface of the body smoothly tapers from the base to the top. Thus, the nozzle has a cross-sectional area that decreases from the base to the top.

The surface of the nozzle is a complex three-dimensional geometric shape generated by connecting lines from every point of the periphery of the substantially annular base to a hypothetical single apex point above the substantially circular surface. Each of the line segments between the periphery of the substantially annular base to the intersection with the substantially circular surface is referred to as a generatrix, particularly a generatrix of the lateral surface.

The nozzle body is bilaterally symmetrical about a plane of symmetry and comprises a longest generatrix (9) and a shortest generatrix (10) being coplanar with each other and the plane of symmetry. The shortest generatrix is a substantially straight line, such as a straight line, substantially parallel with the second central axis. In the context of the present Invention, the term "substantially parallel with the second central axis" is intended to include parallel lines as well as lines that diverge from the second central axis at a small angle of no more than about 10 degrees, such as from about 1 to 10 degrees. In certain embodiments, the shortest generatrix diverges from the second central axis at an angle of from 5 to 10 degrees, such as 5.0, 6.0, 7.0, 8.0, 9.0 or 10.0 degrees, for example 5.3 degrees or 9.7 degrees. The longest generatrix is an inclined concave line. As a result, from the shortest generatrix to the longest generatrix, the surface of the nosepiece smoothly transitions around the second central axis from a substantially flat profile to a concave profile.

An outlet (5) is positioned at the top of the nozzle body within the substantially circular surface (3) through which liquid is able to be discharged. Whilst the outlet may be of any shape, generally the outlet will also be substantially circular. A channel or conduit (6) traverses the nozzle defining a passageway in fluid communication with the substantially annular base and the outlet. Particularly, the nozzle may have a hollow interior in which is formed the channel or conduit structure.

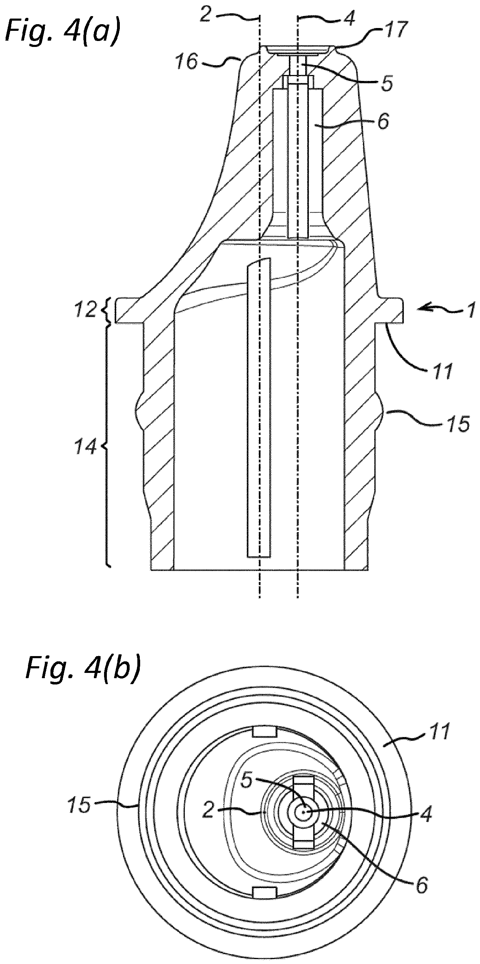

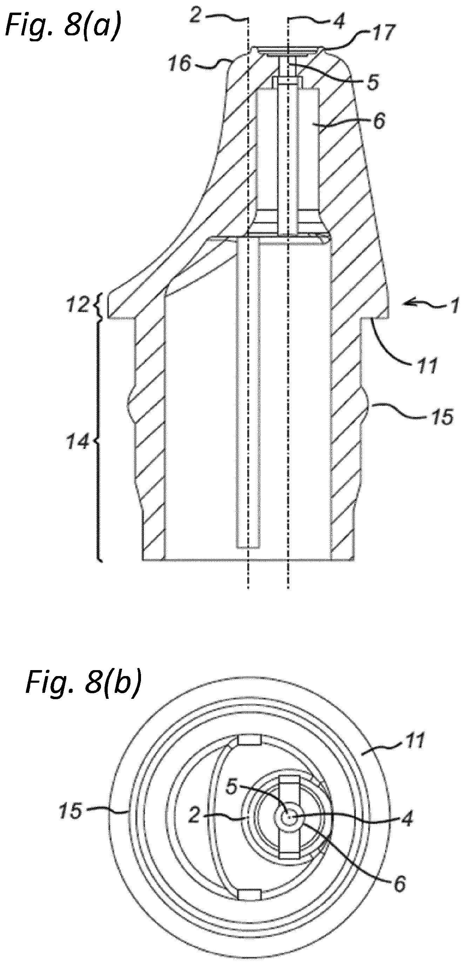

In some embodiments of the Invention, the substantially annular base comprises a shoulder (see for example, Figure 4(a), (11); Figure 8(a), (11)) configured to abut against a surface of a container. Particularly, the shoulder configured to abut against a surface of a container is a surface of a downwardly projecting annular rim (Figure 4(a), (12) and Figure 8(a), (12)).

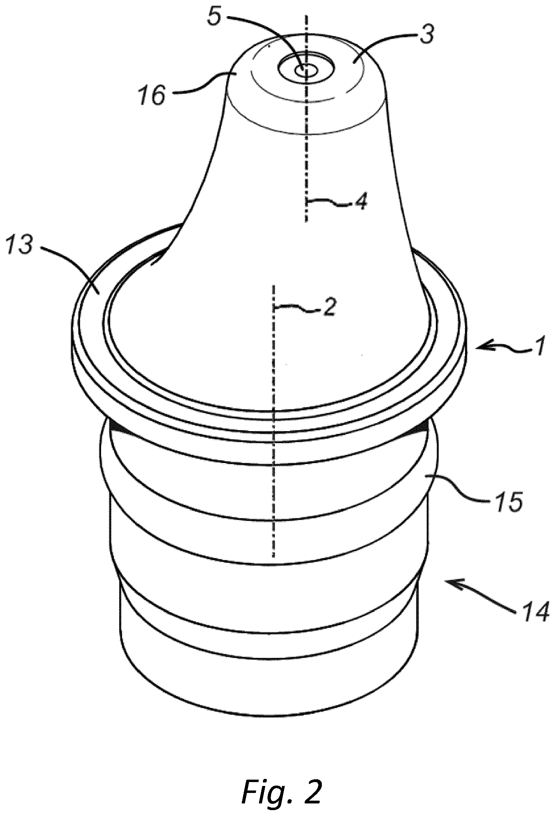

In some embodiments of the Invention, the shoulder configured to abut against a surface of a container is a surface of an annular flange (Figure 2, (13)) that extends radially outwards in a direction substantially perpendicular to the first central axis.

Particularly, the nozzle of the Invention further comprises a substantially cylindrical skirt (14) extending downwardly, particularly axially, from the substantially annular base, configured to attach the nozzle to a container.

In some embodiments, the substantially cylindrical skirt extends downwardly from a position internal of the shoulder and is configured for engagement inside the neck of a container. The engagement may be achieved by "interference fit" where the external dimension of one part slightly exceeds the internal dimension of another part into which it has to fit or by "friction fit" whereby engagement between parts is achieved by friction after the parts are brought together.

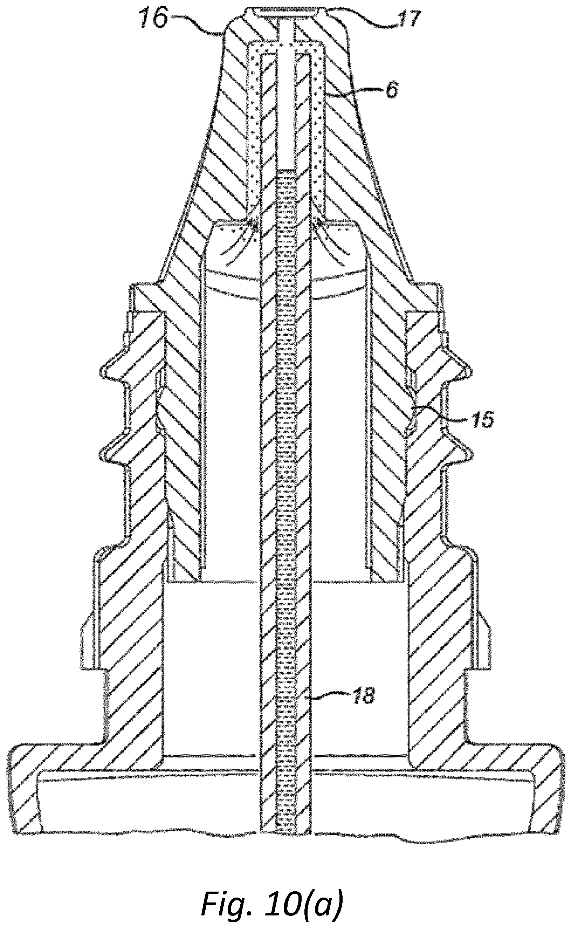

The term "snap fit" is used to describe an engagement means wherein one part has one or more protrusions that fit into one or more indentions on a second part, such that the protrusions 'snap' into the indentions to join the two parts. Particularly, the substantially cylindrical skirt comprises at least one annular gripping rib (15) on its outer surface for sealing and/or gripping engagement with an inner surface of the neck of a container. More particularly the substantially cylindrical skirt comprises at least one annular gripping rib on its outer surface for sealing and/or gripping engagement with at least one annular indentation on the inner surface of the neck of a container (see for example, Figure 10(a)).

The term "screw fit" is used to describe an attachment means wherein one part has helical or advancing spiral threads and the second part has helical grooves of the same size and number of the threads, enabling the two parts to be reversibly joined by twisting into place. In some embodiments, the substantially cylindrical skirt extends downwardly from a position internal of the shoulder and is configured for engagement with the outside of the neck of a container. For example, the substantially cylindrical skirt comprises helical grooves on its inner surface for sealing and/or gripping engagement with corresponding helical threads on an outer surface of the neck of a container.

The nozzle should be comfortable for a user when inserted into a nostril. Particularly, the transition between the lateral surface of the nozzle and the substantially circular surface is a smooth transition, preferably a chamfered edge (16).

In certain embodiments, the outlet is coaxial with the second axis of the substantially circular surface. In other embodiments, the outlet is offset from the second axis of the substantially circular surface.

The nozzle may comprise a substantially circular raised lip (17) extending upwardly from the substantially circular surface around the outlet. The presence of such a raised lip may be advantageous in preventing the outlet becoming obstructed during use. The lip may also engage with a seal, such as a cap, to prevent leakage from a device when not in use. In some embodiments, the substantially circular surface may comprise a recessed area surrounding the outlet, i.e. the outlet is countersunk in the surface. The walls of the recessed area are preferably sloping surfaces and may be curved, particularly concave surfaces.

The nozzle may be connected to a dip tube (18), one end of which is connected with the outlet whereas its other end, in use, opens adjacent to the base of a container. Thus, the nozzle may comprise a dip tube forming part or all of the channel or conduit traversing the nozzle and defining a passageway in fluid communication with the substantially annular base and the outlet. The dip tube will comprise a liquid inlet and a liquid exit. More particularly, the nozzle may comprise a dip tube for conducting liquid upwardly from the bottom of a container to the outlet. Yet more particularly, the dip tube may be attached to and depend from the outlet for conducting liquid upwardly from a container, particularly the bottom of a container, to the outlet. In some embodiments the liquid exit of the dip tube is attached to the nozzle by friction fit and/or adhesive or other bonding agents can be used to further improve the connection.

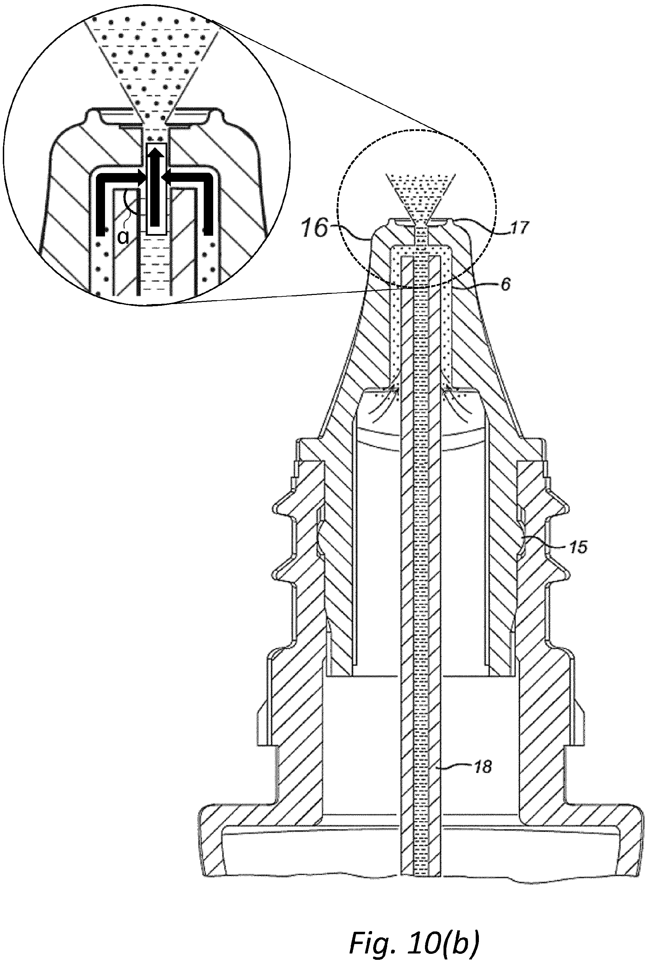

The nozzle may further comprise at least one air channel defining a passageway for directing air from within the nozzle body and/or headspace of a container to at least one orifice in fluid communication with the liquid exit and the outlet (Figure 10(a), (6); Figure 10(b), (6)). In use air directed from within the nozzle body and/or headspace of a container mixes with liquid being conducted upwardly from the container (see for example, Figure 10(b)). The air may help the liquid to break apart and form droplets whilst also helping to project the spray into the nose.

Particularly, the at least one orifice is disposed about the dip tube liquid exit. In some embodiments, the nozzle comprises two air channels and two orifices positioned in a circumferential arrangement around the dip tube. In other embodiments, the nozzle comprises three air channels and three orifices positioned in a circumferential arrangement around the dip tube. In yet other embodiments, the nozzle comprises four air channels and four orifices positioned in a circumferential arrangement around the dip tube. Particularly, the air emitted from the orifice(s) is emitted at an angle (a) of from 0 to 90 degrees with respect to the direction of movement of the liquid towards the outlet (Figure 10(b), (a)). More particularly, the air emitted from the orifice(s) is emitted at an angle of from 45 to 90 degrees with respect to the direction of movement of the liquid towards the outlet, for example, 45, 50, 55, 60, 65, 70, 75, 80, 85 or 90 degrees. Yet more particularly, the air emitted from the orifice(s) is emitted at an angle of about 90 degrees with respect to the direction of movement of the liquid towards the outlet. In certain embodiments, the air emitted from the orifice(s) may be emitted at an angle of from 85 to about 90 degrees with respect to the direction of movement of the liquid towards the outlet, such as 85, 86, 87, 88, 89 or 90 degrees. In certain other embodiments, the air emitted from the orifice(s) may be emitted at an angle of greater than 90 degrees with respect to the direction of movement of the liquid towards the outlet, for example from 91 to 100 degrees, such as 91, 92, 93, 94, 95, 96, 97, 98, 99 or 100 degrees. In yet other embodiments, the air emitted from the orifice(s) may be emitted at an angle of from 85 to 95 degrees with respect to the direction of movement of the liquid towards the outlet.

Particularly, an inner wall of the at least one air channel is defined and bounded by an outer wall of the dip tube (as shown in Figures 10 (a) and (b)).

Particularly, the nozzle is injection-moulded as a one-piece component from a thermo-plastics material. More particularly, the nozzle is injection-moulded as a one-piece component from low-density polyethylene (LDPE). In some embodiments, the dip tube is an integral part of the nozzle. In other embodiments, the body of the nozzle is injection-moulded as a one-piece component from LDPE and the dip tube is a separate component that is attached in a further process step.

The nozzles of the Invention are for attachment to a squeeze actuated container or bottle. Squeeze containers are usually formed from a resiliently deformable, pliable material such that when the walls of the container are deformed, the contents of the container, usually a liquid, are forced up a dip tube, into the nozzle and then out of the outlet. Preferably the container can be easily deformed by squeezing it with one hand. Generally, the container comprises two deformable regions arranged opposite each other to facilitate squeezing by a hand gripping the bottle. The deformability of the container may be limited to at least two predetermined regions, so that if the other regions of the container are pressed there is no discharge of liquid spray. Containers may be formed by any suitable process such as, thermo-forming, blow-moulding, injection-moulding, injection stretch blow moulding and the like. Preferably, the container is formed by blow-moulding. More preferably, the contained is formed by blow-moulding from LDPE.

Thus, there is also provided a squeeze-actuated device for intranasal administration of a liquid comprising: a container defining an internal volume intended to hold the liquid, the container having at least one flexible wall and a nozzle of the Invention, as described supra. In some embodiments the squeeze-actuated device for intranasal administration of a liquid comprises: a container defining an internal volume intended to hold the liquid, the container having at least one flexible wall and a neck having an opening with an inner surface; and a nozzle of the Invention wherein engagement of the outer surface of the substantially cylindrical skirt with the inner surface of the neck forms a friction-fit connection between the container and the nozzle. A closure cap may also be provided to be screwed onto the neck of the container over the nozzle. Preferably, the closure cap is formed from high-density polyethylene (HDPE).

The squeeze-actuated device of the Invention is particularly suitable for use with over the counter (OTC) liquid formulations. Thus, the squeeze-actuated device further comprises a liquid, more particularly a liquid formulation. Liquids and liquid formulations are preferably pharmaceutically acceptable. The liquid formulation may comprise an active pharmaceutical ingredient or API. In certain embodiments, the API is an imidazoline nasal decongestant. In certain embodiments the liquid formulation comprises Xylometazoline or Oxymetazoline. Preferably, the liquid formulation comprises Oxymetazoline. In other embodiments, the liquid is saline or sterile water.

The container will have a headspace above the liquid. As used herein, the term "headspace" generally means that region of the interior of the container above the level of any liquid contained therein. In the context of the present Invention, the headspace will be an airspace, particularly filled with air, more particularly atmospheric air.

In use, the manual squeezing of the container will bring generally opposing portions of the deformable container sidewall closer together, causing an increase in the interior pressure. Since there is headspace containing air above the liquid, as opposing portions of the container sidewall are squeezed together the interior volume of the container is reduced, generating interior pressure that causes the air to find an exit path of least resistance. This interior pressure also causes the liquid to find an exit path of least resistance. Thus, this increase in the interior pressure creates both an air flow in the air channel(s) and a flow of the liquid upwardly through the dip tube (Figure 10(a)). These flows of air and liquid are combined and pushed through the outlet thereby creating a spray of liquid out of the nozzle causing a quantity of atomized liquid to be administered into the nasal cavity (Figure 10(b)). When the squeezing force on the container is released, the container substantially returns to its starting shape. This in turn creates an internal suction force which pulls, in atmospheric air until the interior pressure is restored to generally atmospheric pressure.

### GENERAL

The term "comprising" encompasses "including" e.g. a composition "comprising" X may include something additional e.g. X + Y. In some implementations, the term "comprising" refers to the inclusion of the indicated active agent, such as recited polypeptides, as well as inclusion of other active agents, and pharmaceutically acceptable carriers, excipients, emollients, stabilizers, etc., as are known in the pharmaceutical industry. In some implementations, the term "consisting essentially of" refers to a composition, whose only active ingredient is the indicated active ingredient(s), for example ibuprofen-arginine salt, however, other compounds may be included which are for stabilizing, preserving, etc. the formulation, but are not involved directly in the therapeutic effect of the indicated active ingredient. Use of the transitional phrase "consisting essentially" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. Where methods refer to process steps, for example as (a), (b), (c), etc., these are intended to be sequential, i.e., step (c) follows step (b) which is preceded by step (a).

All references or patent applications cited within this patent specification are incorporated by reference herein. In order that this invention may be better understood, the following examples are set forth. These examples are for purposes of illustration only and are not to be construed as limiting the scope of the invention in any manner.

### Examples

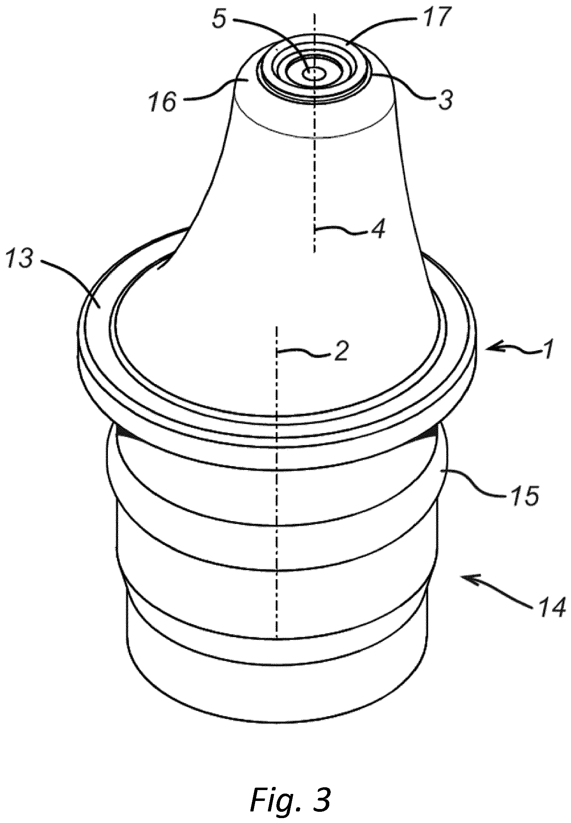

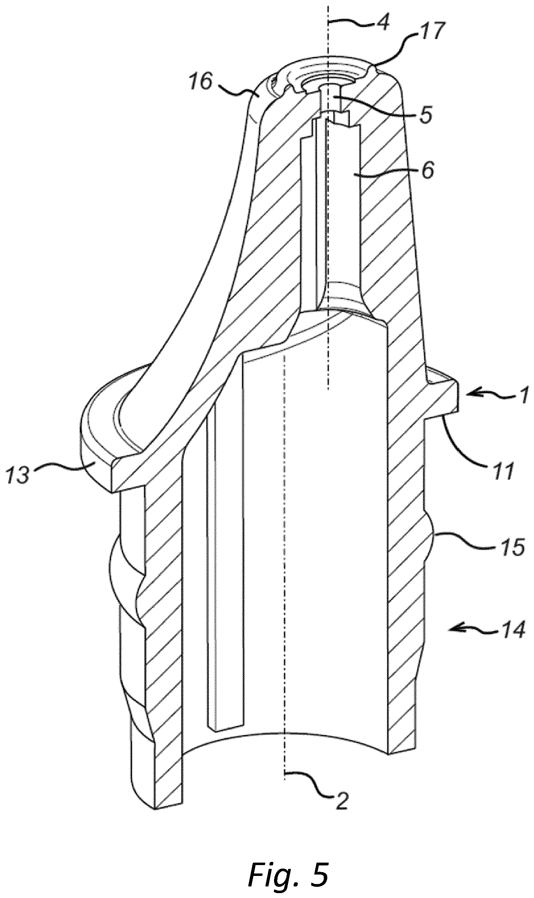

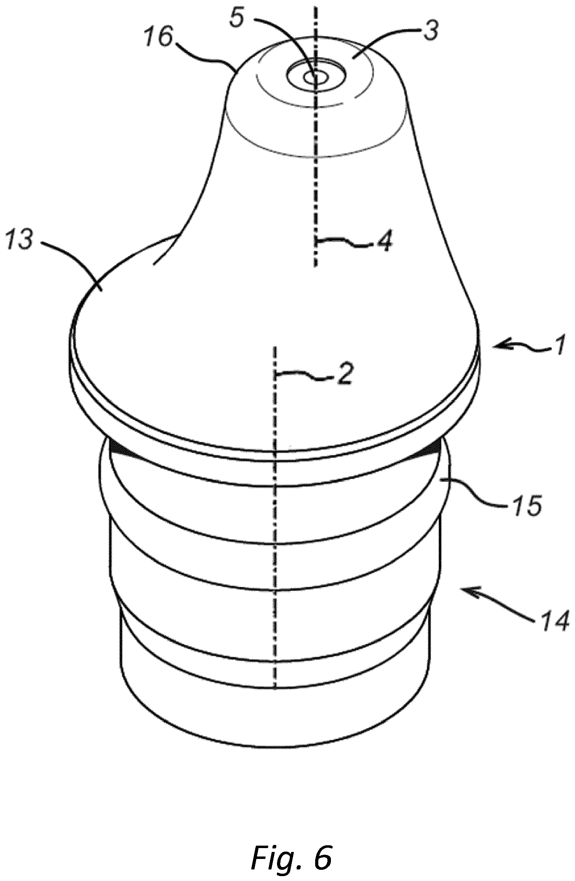

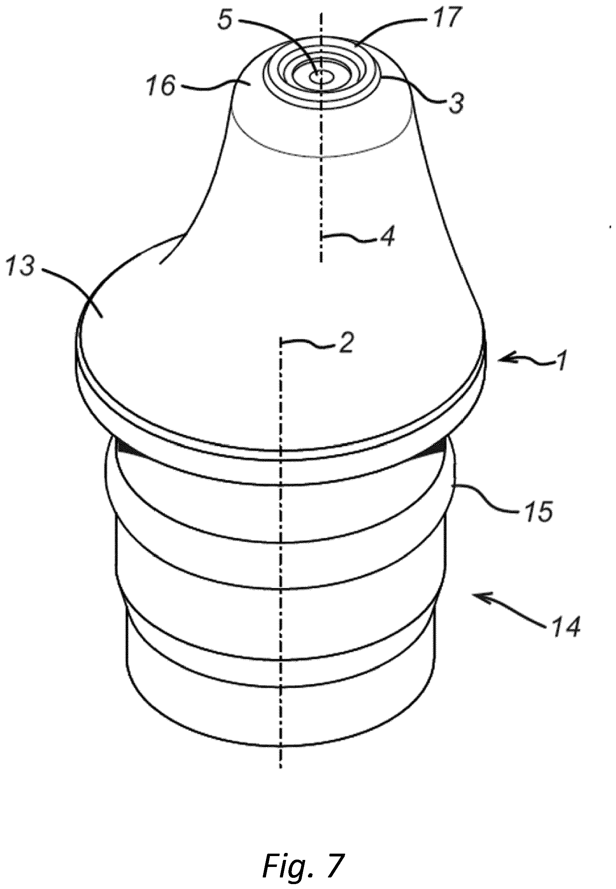

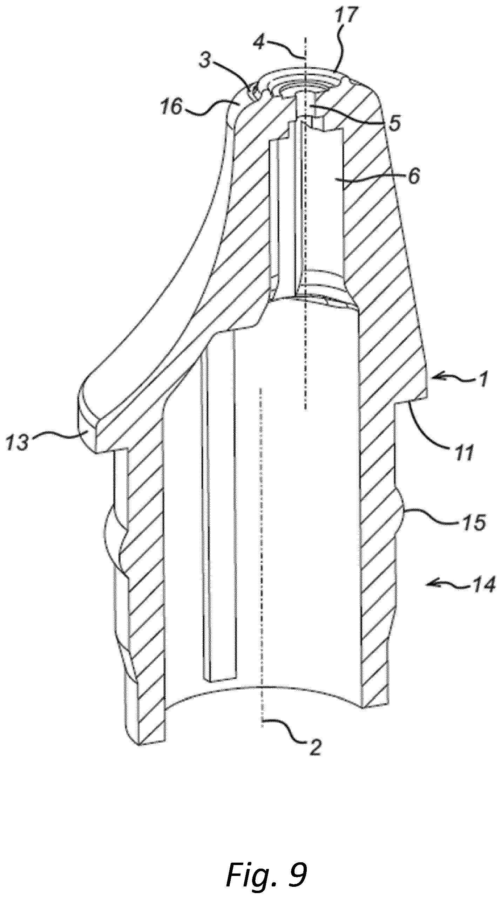

Figures 2 to 5 illustrate two variants of friction fit' nozzle in accordance with the present invention each of which comprises an annular flange (13) at the base. The nozzle of Figure 2 comprises a substantially annular base (1) comprising an annular flange (13) having a central axis (2). The top of the nozzle comprises a substantially circular surface (3) also having a central axis (4). A chamfered edge (16) provides a smooth transition from the outer surface to the top. The spray outlet of the nozzle (5) sits within a recess in the substantially circular top surface. As can be seen, the outer diameter of the base is larger than thar if the top surface such that the body tapers from base to top. In this embodiment, the nozzle comprises a substantially cylindrical skirt (14) with an annular gripping rib (15) for engaging with the neck of a container (not shown). Figure 3 provides a further variant that comprises a substantially circular raised lip (17) extending upwardly from the substantially circular surface around the recessed outlet. The presence of the raised lip prevents the outlet becoming obstructed during use. The lip is designed to also engage with a seal in a cap (not shown), thereby preventing leakage from an assembled device when not in use. Figure 4(a) is a cutaway of the nozzle showing the shoulder (11) that rests against a surface of a container (not shown). Part of a dip tube is shown extending from the outlet (5) through part of the channel (6). Figure 4(b) provides a view from underneath the nozzle showing two internal strengthening supports, appearing as two rectangles at 0 and 180 degrees. Two air-channels are also shown positioned above and below the outlet (5). Figures 6 to 9 provide two further variants of the nozzles described above that lack the annular flange.

Figure 10(a) shows a cutaway of a nozzle of the Invention installed in the neck of a container (full container not shown). In use, the manual squeezing of the container brings opposing portions of the container sidewall closer together, causing an increase in the interior pressure. Since there is headspace containing air above the liquid, as opposing portions of the container sidewall are squeezed together the interior volume of the container is reduced, generating interior pressure that causes the air to find an exit path of least resistance. This interior pressure also causes the liquid to find an exit path of least resistance. This increase in the interior pressure creates an air flow in the air channels (arrows) and a flow of the liquid upwardly through the dip tube (18). These flows of air and liquid meet and are combined and pushed through the outlet thereby creating a spray of liquid out of the nozzle causing a quantity of atomized liquid to be administered into the nasal cavity (Figure 10(b)). When the squeezing force on the container is released, the container substantially returns to its starting shape. This in turn creates an internal suction force which pulls, in atmospheric air until the interior pressure is restored to generally atmospheric pressure.

## Claims

1. A nozzle for intranasal administration of a liquid from a squeeze-actuated device, comprising:
a body having the shape of a truncated oblique cone having a substantially annular base with a first central axis and a substantially circular surface at the top with a second central axis;
an outlet positioned at the top of the body within the substantially circular surface;
a channel traversing the nozzle defining a passageway in fluid communication with the substantially annular base and the outlet;
wherein the outer diameter of the substantially annular base is larger than the outer diameter of the substantially circular surface and the second central axis of the top is parallel to and offset from the first central axis of the base and the surface of the body smoothly tapers from the base to the top;
wherein the body is bilaterally symmetrical about a plane of symmetry the body comprising a longest generatrix and a shortest generatrix being coplanar with each other and the plane of symmetry; and
wherein the shortest generatrix is a substantially straight line parallel with the second central axis and the longest generatrix is an inclined concave line such that the surface of the nosepiece smoothly transitions from a substantially flat to a concave profile around the second central axis.

2. The nozzle of Claim 1, wherein the substantially annular base comprises a shoulder configured to abut against a surface of a container.

3. The nozzle of Claim 2, wherein (i) the shoulder configured to abut against a surface of a container is a surface of a downwardly projecting annular rim or wherein (ii) the shoulder configured to abut against a surface of a container is a surface of an annular flange that extends radially outwards in a direction substantially perpendicular to the first central axis.

4. The nozzle of any one of Claims 2 or 3, further comprising a substantially cylindrical skirt extending downwardly from the substantially annular base, configured for attachment to a container.

5. The nozzle of Claim 4, wherein the substantially cylindrical skirt extends downwardly from a position internal of the shoulder and is configured for engagement inside the neck of a container.

6. The nozzle of Claim 5, wherein the substantially cylindrical skirt comprises at least one annular gripping rib on its outer surface for sealing and/or gripping engagement with an inner surface of the neck of a container.

7. The nozzle of any preceding claim, wherein the transition between the surface of the nosepiece and the substantially circular surface is a chamfered edge.

8. The nozzle of Claim 7, wherein (i) the outlet is coaxial with the second axis of the substantially circular surface or wherein (ii) the outlet is offset from the second axis of the substantially circular surface.

9. The nozzle of any preceding claim, comprising a substantially circular raised lip extending upwardly from the substantially circular surface around the outlet.

10. The nozzle of Claim 8 or 9, which comprises a dip tube having a liquid inlet and a liquid exit, depending from the outlet for conducting liquid upwardly from a container to the outlet.

11. The nozzle of Claim 10, further comprising at least one air channel defining a passageway for directing air from within the body to at least one orifice in fluid communication with the liquid exit and the outlet, wherein in use air directed from within the body mixes with liquid being conducted upwardly from the container.

12. The nozzle of Claim 11, wherein the at least one orifice is disposed about the dip tube liquid exit.

13. The nozzle of Claim 11 or 12, comprising two air channels and two orifices positioned in a circumferential arrangement around the dip tube.

14. The nozzle of Claim 11, 12 or 13, wherein the air emitted from the orifice(s) is emitted at an angle of from 0 to 90 degrees, particularly from 45 to 90 degrees, with respect to the direction of movement of the liquid towards the outlet.

15. The nozzle of any one of Claims 11 to 14, wherein an inner wall of the at least one air channel is defined and bounded by an outer wall of the dip tube.

16. A squeeze-actuated device for intranasal administration of a liquid comprising: a container defining an internal volume intended to hold the liquid, the container having at least one flexible wall and a nozzle according to any one of Claims 1 to 15.

17. A squeeze-actuated device for intranasal administration of a liquid comprising:
a container defining an internal volume intended to hold the liquid, the container having at least one flexible wall and a neck having an opening with an inner surface;
and a nozzle according to any one of Claims 4 to 15 wherein engagement of the outer surface of the substantially cylindrical skirt with the inner surface of the neck forms a friction-fit connection between the container and the nozzle.

18. The squeeze-actuated device of Claim 16 or 17 which further comprises a liquid, particularly a liquid formulation of an active pharmaceutical ingredient, more particularly a liquid formulation comprising Oxymetazoline or Saline.
